**Europäisches Patentamt**

**European Patent Office**

**Office européen des brevets**

(19)

(11) Veröffentlichungsnummer: **0 117 894**
**B1**

(12) **EUROPÄISCHE PATENTSCHRIFT**

(45) Veröffentlichungstag der Patentschrift:
08.11.89

(51) Int. Cl.⁴: **A 61 B 1/00**

(21) Anmeldenummer: 83109117.8

(22) Anmeldetag: 15.09.83

(54) Endoskop mit medizinischem Greifinstrument.

(30) Priorität: 08.02.83 DE 8303342 U

(73) Patentinhaber: Storz-Endoskop GmbH, Kreuzgutweg 22, CH-8207 Schaffhausen (CH)

(43) Veröffentlichungstag der Anmeldung:
12.09.84 Patentblatt 84/37

(72) Erfinder: Storz, Karl, Auf dem Schildrain 39, D-7200 Tuttlingen (DE)

(45) Bekanntmachung des Hinweises auf die Patenterteilung:
08.11.89 Patentblatt 89/45

(74) Vertreter: Wenzel, Joachim, Dipl.-Ing., Hauptmannsreute 46, D-7000 Stuttgart 1 (DE)

(84) Benannte Vertragsstaaten:
AT BE CH DE FR GB IT LI NL SE

(56) Entgegenhaltungen:
DE-A- 2 628 555
DE-A- 2 829 159
DE-A- 2 927 726
DE-A- 3 012 447

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Endoskop nach dem Oberbegriff des Anspruchs 1.

Es ist bereits ein derartiges Endoskop mit einer längsverschieblichen Drahtschlinge bekannt, bei der die Verbindung des teleskopischen Sehrores mit der Drahtschlinge über ein Getriebe in der Weise vorgesehen ist, daß die Verschiebung des Sehrohres in einem geringeren Ausmaß als die Verschiebung der Drahtschlinge erfolgt. Dadurch wird die Verwendung eines teleskopischen Sehrohres mit einem weiten Blickfeld im wesentlichen ohne Behinderung der Sicht durch den Rand des Endoskopschaftes ermöglicht (DE-OS 2 628 555).

Es ist weiter eine Sonde zum Entfernen von Harnleitersteinen mit einer am Vorderende eines Katheterschlauches angeordneten Greiferanordnung bekannt, welche über ein innerhalb des Katheterschlauches geführtes Betätigungskabel geöffnet und geschlossen werden kann, wobei auf dem Vorderende des Katheterschlauches eine längsverschiebliche Hülse angeordnet ist, welche eine aufblasbare, radial erweiterbare Manschette trägt. Hierbei ist auch ein bildübertragender Lichtleiter konzentrisch in einer Kugel vorgesehen. Durch Zurückziehen der Kugel werden die Greifarme federnd auseinander bewegt. Dies zeigt aber den Nachteil, daß der Öffnungswinkel der Greifarme nur verhältnismäßig klein sein kann, so daß nur entsprechend kleine Steine gefaßt werden können. Außerdem ist die Bildübertragung durch die flexiblen Lichtleiter wesentlich schlechter als bei einem starren Sehrohr, auch läßt sich eine derartige Sonde in manchen medizinischen Fällen nicht verwenden, wo ein starres Endoskop erforderlich ist (DE-OS 2 927 726).

Der Erfindung liegt die Aufgabe zugrunde, ein starres Endoskop dieser Art so zu verbessern, daß die Greifarme immer im Sichtbereich des Sehrohres liegen und Platz eingespart wird, so daß der Außendurchmesser des Endoskopschaftes möglichst klein ausgeführt werden kann.

Außerdem soll erreicht werden, daß der Öffnungswinkel der Greifarme groß sein kann, so daß auch größere Steine erfaßt werden können.

In weiterer Ausgestaltung der Erfindung sind die kennzeichnenden Merkmale des Anspruchs 2 vorteilhaft. Durch diese Nachführung des Sehrohres wird unter anderem erreicht, daß die Gefahr beseitigt ist, daß das Objektiv etwa durch einen Stein beschädigt werden kann, der von den Greifarmen zurückgezogen wird. Es wird auch genügend Platz am patientennahen Ende zur Aufnahme solcher Steine oder Fremdkörper im Endoskop geschaffen.

Weitere Vorteile und Einzelheiten der Erfindung ergeben sich aus der nun folgenden Beschreibung einiger Ausführungsformen unter Hinweis auf die Zeichnung. In dieser zeigen:

Fig. 1 eine Seitenansicht mit stark verkürztem Endoskopschaft auf das gesamte Instrument;

Fig. 2 eine Seitenansicht auf einen Teil des Instrumentes mit demontiertem Endoskopschaft;

Fig. 3 eine Seitenansicht auf eine erste Ausführungsform der Betätigungshülse;

Fig. 4 eine Ansicht wie Fig. 3 auf eine weitere Ausführungsform;

Fig. 5 eine teilweise Ansicht auf die Ausführungsform nach Fig. 4, demgegenüber jedoch um 90° gedreht und

Fig. 6 eine Ansicht wie Fig. 2 jedoch nach der Demontage der Betätigungshülse.

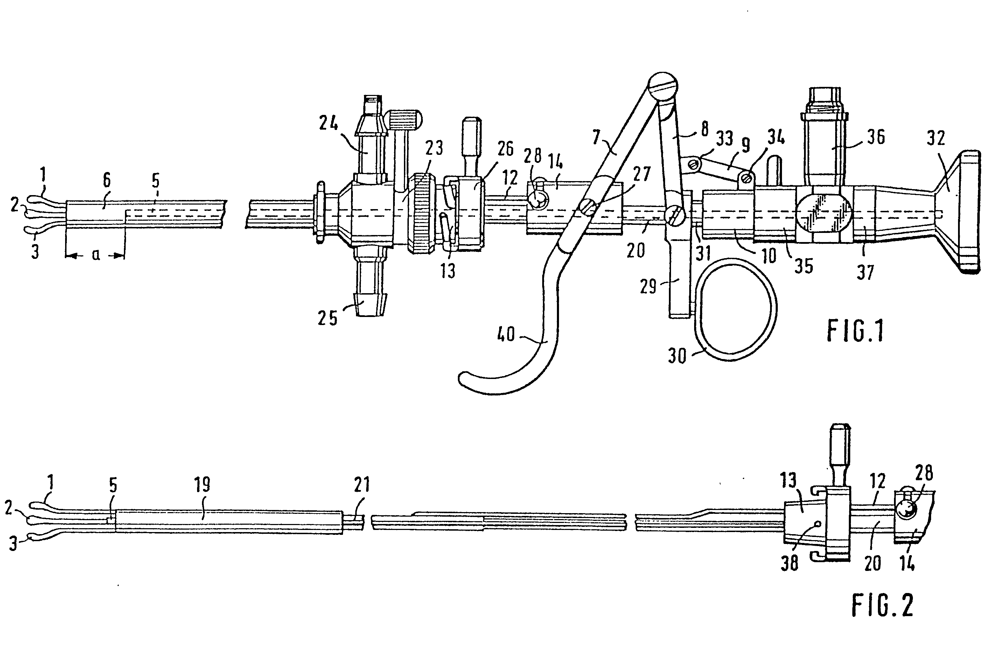

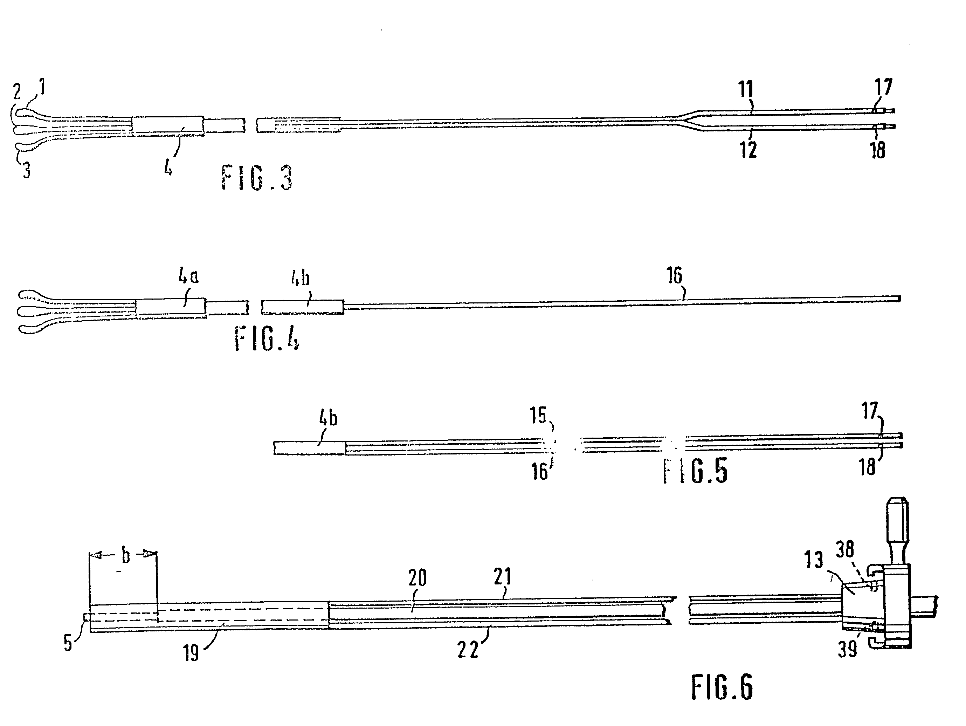

Fig. 1 zeigt das Instrument in einer Zwischenlage, bei der die Greifarme 1, 2, 3 nicht vollständig ausgefahren sind, sie sind aber auch nicht vollständig in den Endoskopschaft 6 eingezogen, sondern sie ragen noch ein Stück nach links über das Ende des Endoskopschaftes 6 hinaus.

Wie man sieht, federn sie nach außen und sind völlig zentrisch zu dem Endoskopschaft 6 angeordnet.

Innerhalb des Schaftes 6 befinden sich mehrere Hülsen, die aber der Übersichtlichkeit wegen weggelassen wurden. Durch unterbrochene Linien wurde lediglich das sehr dünne Sehrohr 5 eingezeichnet, dessen distales Ende in dieser Stellung um den Abstand a vom distalen Ende des Schaftes 6 entfernt ist. Beim weiteren Ausschieben nach links der Greifarme wird jedoch das Objektiv des Sehrohres 5 ebenfalls weiter nach links verschoben, wie später noch erläutert wird.

Weiter rechts sieht man den Schaftverschluß 13 mit dem Bajonettring 23 herkömmlicher Art. Es ist ein Vorteil dieser Ausführungsform, daß ein herkömmlicher Endoskopschaft mit den Spül-Anschlüssen 24, 25 Verwendung finden kann. Der Bajonettverschlußring 26 gehört bereits zu dem Schaftverschluß 13, wie später noch erläutert wird.

Weiter rechts sieht man den Draht 12 der Betätigungshülse 4 und darunter die ortsfeste Hülse 20 für das Sehrohr 5. Weiter rechts ist der Schlitten 14 zu sehen, der auf dem ortsfesten Rohr 20 gleitbar durch die beiden Hebel 7 und durch Federkraft nach links geschoben wird. Derartige Hebelgestänge sind für sich bekannt und müssen daher nicht im einzelnen dargestellt werden. Der Hebel 7 endet unten in einer Handhabe und ist um die Schraube 27 schwenkbar gelagert. Links oben ist an dem Schlitten 14 eine Arretiervorrichtung 28 zu sehen, durch die die Betätigungsdrähte 11 und 12 lösbar in dem Schlitten 14 befestigt sind. Der Hebel 8 ist oben an dem senkrechten ortsfesten Teil 29 gelenkig gelagert, an dem unten die Handhabe 30 zur Einführung des Daumens befestigt ist.

Die Hülse 10 ist zusammen mit dem ganzen übrigen rechts davon liegenden Teil des Endoskopes mit dem Okular 32 auf der ortsfesten Hülse 31 durch den Hebel 5 bewegbar, der an seinen Enden 33, 34 an der Hülse 10 bzw. dem Hebel 8 angelenkt ist. Wenn der Schieber 14 sich nach links bewegt, dann folgt die Hülse 10 dieser Bewegung nach links in verkleinertem Maßstab. Derartige Getriebe sind für sich bekannt und müssen daher nicht im einzelnen beschrieben werden.

Durch den Bajonettring 35 ist das Okular-Teil 32 mit dem Lichtleiteranschluß 36 mit der Hülse 10 verbunden, die man auch mit «Schieber» bezeichnen könnte. Der Bajonettring 35 sitzt ständig auf der Hülse 10. Durch Drehen desselben läßt sich das Okularteil 37 zusammen mit dem Sehrohr 5 lösen und nach rechts herausziehen.

Die Einzelheiten des Erfindungsgegenstandes werden anhand der Einzelteile beschrieben, wie sie sich bei der Demontage ergeben. Zunächst wird der Endoskopschaft 6 durch Drehen des Bajonettverschlußringes 26 gelöst und nach links abgezogen. Danach verbleibt das in der Fig. 2 nur teilweise dargestellte Restinstrument. Hierin sieht man links wieder die Greiferarme 1, 2, 3 in etwas größerer Länge, deren unterstes Teil allerdings von einer Hülse 19 ummantelt ist, die durch zwei Drähte 21 und 22, von denen nur der Draht 21 zu sehen ist, mit dem Schaftverschluß 13 fest verbunden ist. Links an der Hülse 19 sieht man das distale Ende mit dem Objektiv des Sehrohres 5, das einen wesentlich kleineren Durchmesser hat.

Diese Hülse 19 dient lediglich dazu, die Arme 1, 2 und 3 zu betätigen, wenn der Endoskopschaft 6, wie hier dargestellt, fehlt. Es besteht nämlich nun die Möglichkeit, die Arme nach rechts in die Hülse 19 hineinzuziehen. Sie müssen aber nicht vollständig hineingezogen werden, wie das bezüglich des Schaftes 6 der Fall ist, sondern nur so weit, daß der Arzt das Instrument nach Fig. 2 unter Betätigung des Hebels 7 mühelos in den Schaft 6 einführen kann.

Der weiter rechts sichtbare Betätigungsdraht 12 gehört mit einem weiteren dahinter liegenden Draht 11 zu einer hier nicht sichtbaren Betätigungshülse 4, die von der Hülse 19 ummantelt ist. Wenn man in Fig. 2 die Arretiervorrichtung 28 löst, lassen sich die erwähnten Drähte 11 und 12 durch den Schaftverschluß 13 nach links zusammen mit den Greiferarmen 1, 2 und 3 sowie der erwähnten Betätigungshülse 4 herausziehen.

In Fig. 3 sieht man nun diese Betätigungshülse 4, an die rechts die beiden Betätigungsdrähte 11, 12 aufgelötet sind. Sie befinden sich zunächst im Bereich der Hülse 4 dicht nebeneinander und sind auch zusammen gelötet. Erst weiter rechts sind sie gabelförmig voneinander getrennt und zeigen an ihren Enden Einkerbungen 17, 18 zur Einführung in die Arretiervorrichtung 28. Es handelt sich somit um einen lösbaren Steckverschluß. Die Trennung der beiden Drähte 11, 12 ist erforderlich, um zwei verschiedene Gleitlager in dem Schaftverschluß 13 ausbilden zu können, es kann sich z.B. um Kunststoff-Lager in dem Verschluß 13 handeln.

Fig. 4 zeigt eine weitere Ausführungsform der Betätigungshülse 4. Diese ist aus zwei Hülsen 4a und 4b gebildet, die miteinander verbunden sind. Dies ist erforderlich, weil die Hülse 4a aus Federstahl gefertigt sein muß, wenn die Arme 1, 2, 3 einstückig mit der Hülse 4a ausgebildet sein sollen. Dagegen ist die Hülse 4b aus härterem Stahl gefertigt, weil sie lediglich zur Betätigung der Hülse 4a vorgesehen ist. Den rechten Teil der Hülse 4b hat man beidseitig so bearbeitet, daß in dieser Ansicht lediglich die Verlängerung 16 stehen geblieben ist, hinter der die Verlängerung 15 liegt.

Fig. 5 zeigt das rechte Ende der Hülse 4b um 90° gedreht. Hier sieht man, daß infolge der Bearbeitung zwei Teile 15, 16 im Abstand zueinander stehen geblieben sind. Es besteht bei einer dritten Ausführungsform auch noch die Möglichkeit, einen der beiden Teile 15 oder 16 zu entfernen, sodaß lediglich eines der beiden Teile stehen bleibt, welches zur Betätigung der Hülse 4b ausreicht. Am rechten Ende sind dann wieder die Einkerbungen 17 und 18 für den Verschluß 28 vorgesehen wie gemäß Fig. 3.

Die Bearbeitung der Hülse 4b kann z.B. durch Schleifen oder Fräsen der vollständig ausgebildeten Hülse erfolgen, sodaß dann die Teile 15, 16 stehen bleiben.

Fig. 6 zeigt das Restteil, was übrig geblieben ist, nachdem die Betätigungshülse 4 in der zuvor erwähnten Weise demontiert wurde. Es handelt sich also um die Darstellung nach Fig. 2 ohne die Betätigungshülse 4.

Links sieht man wieder das Sehrohr 5 ein wenig die Hülse 19 überragen. Die beiden Drähte 21, 22 sind in diesem Falle innerhalb der Hülse 19 angelötet. Zwischen diesen beiden Drähten liegt die schon erwähnte ortsfeste Hülse für das Sehrohr 20. Durch die unterbrochenen Linien ist angedeutet, daß diese Hülse 20 um einen Abstand b vor dem linken Ende der Hülse 19 bereits endet, damit hier mehr Platz für die Aufnahme der Greiferarme 1, 2, 3 verbleibt. Wie vorhin schon erwähnt, müssen diese nicht vollständig in diese Hülse 19 eingezogen werden.

In dem Schaftverschluß 13 sind durch unterbrochene Linien die beiden Nieten 38 und 39 angedeutet, von denen einer durch voll ausgezogene Linien auch in Fig. 2 sichtbar ist. Hieraus mag man auch erkennen, daß die Anordnung nach Fig. 3 gegenüber der nach Fig. 2 um 90° gedreht dargestellt ist. Somit ist die Hülse 19 durch die Drähte 21, 22 nicht lösbar mit dem Schaftverschluß 13 verbunden.

Im nachfolgenden wird die Wirkungsweise kurz erläutert, soweit nicht schon geschehen. Wenn die Handhabe 40 des Hebels 7 unter dem Druck einer Feder nach links bewegt wird, werden die Greifer 1, 2, 3 nach links weiter ausgefahren, wobei sich das Objektiv des Sehrohres ebenfalls weiter nach links bewegt, weil es durch die Hülse 10 in einem veränderten Maßstab der Bewegung des Schiebers 14 nach links folgt.

Wenn nun die Handhabe 40 nach rechts bewegt wird, läuft der Schieber 14 nach rechts und bewegt durch die Drähte 12 die Betätigungshülse 4 mit den Greiferarmen 1, 2, 3 nach rechts und zieht diese in den Endoskopschaft 6 hinein. Somit werden die Greiferarme durch das Schaftende 6 betätigt. Dabei bewegt sich auch das Sehrohr 5 weiter nach rechts, um den Raum mit dem Abstand a nicht nur für die Greiferarme zu schaffen, sondern auch für mögliche Steine oder sonstige Gegenstände, die zuvor unter Sichtbeobachtung erfaßt worden sind. Bei der weiteren Bewegung nach rechts wird das Sehrohr 5 sogar noch hinter das Ende der Hülse 19 in die Hülse 20 hineingezogen, sodaß eine Beschädigung des Objektives 5 durch einen Nierenstein oder dergleichen nunmehr ausgeschlossen erscheint.

Die Erfindung ist nicht auf die dargestellten Ausführungsformen beschränkt. Beispielsweise ist auch ohne weiteres möglich, wie schon oben erwähnt, ganz ohne die Hülse 19 mit den Drähten 21, 22 zu arbeiten. Diese dienen lediglich der leichteren Handhabung beim Einführen in den Schaft. Auch die Hülse 20 muß sich nicht unbedingt in den Endoskopschaft hinein fortsetzen.

Der Hauptvorteil besteht indessen darin, daß die Greiferarme sich stets unter guter Sichtbeobachtung durch das Sehrohr befinden. Dadurch, daß dieses nachgeführt wird, ist die Beobachtung in jeder Lage der Greiferarme optimal. Außerdem ergibt sich gegenüber dem Stand der Technik noch der Vorteil, daß das Sehrohr durch Lösen des Bajonettringes 35, wie schon erwähnt, sehr leicht für sich allein demontiert werden kann. Außerdem kann das gesamte Gerät leicht aus dem Endoskopschaft herkömmlicher Bauart herausgezogen werden.

## Patentansprüche

1. Endoskop mit einem medizinischen Instrument, das im Sichtbereich des starren Sehrohres des Endoskops durch ein Gestänge manipulierbar ist, dadurch gekennzeichnet, daß konzentrisch um das Sehrohr (5) eine Betätigungshülse (4) vorgesehen ist, daß am Ende dieser Betätigungshülse (4) mehrere konzentrisch zueinander angeordnete federnde Greifarme (1, 2, 3) befestigt sind, wobei die Betätigungshülse (4) konzentrisch und axial verschiebbar innerhalb eines Endoskopschaftes (6) angeordnet ist, so daß die Greifarme in den Endoskopschaft (6) hineingezogen und aus diesem nach außen seitlich federnd ausgeschoben werden können.

2. Endoskop nach Anspruch 1, dadurch gekennzeichnet, daß das Gestänge (7, 8) zur Bewegung der Betätigungshülse (4) über ein Gradschubhebelgestänge (9, 10) und einen axial bewegbaren Schlitten (14) mit dem Sehrohr (5) so verbunden ist, daß die Verschiebung des Sehrohres (5) in einem unterschiedlichen Ausmaß gegenüber der Verschiebung der Betätigungshülse erfolgt.

3. Endoskop nach Anspruch 1, dadurch gekennzeichnet, daß die Betätigungshülse (4) mit einem oder mehreren Betätigungselementen (11, 12; 15, 16) verbunden ist, die den Schaftverschluß (13) in einer Gleitführung durchdringen und in dem axial bewegbaren Schlitten (14) des Gestänges lösbar befestigt sind.

4. Endoskop nach Anspruch 1, dadurch gekennzeichnet, daß die Betätigungselemente als Drähte (11, 12) ausgebildet sind, die mit der Betätigungshülse (4) verlötet sind.

5. Endoskop nach Anspruch 4, dadurch gekennzeichnet, daß die beiden Drähte (11, 12) zur Führung in dem Schaftverschluß (13) vor Eintritt in denselben gabelförmig voneinander getrennt sind.

6. Endoskop nach Anspruch 3, dadurch gekennzeichnet, daß die Betätigungselemente als ein oder mehrere einstückige Verlängerungen (15, 16) der Betätigungshülse (4) mit kleinerem Querschnitt als dieselbe ausgebildet sind.

7. Endoskop nach Anspruch 1, dadurch gekennzeichnet, daß eine Außenhülse (19) die Betätigungshülse (4) ummantelnd durch Befestigungselemente (21, 22) mit dem Schaftverschluß (13) verbunden ist.

8. Endoskop nach Anspruch 1, dadurch gekennzeichnet, daß eine durchgehende ortsfeste Hülse (20) zur Führung des Sehrohres (5) angeordnet ist, die in einem Abstand (b) vor dem distalen Ende der Außenhülse (19) endet.

## Claims

1. Endoscope with a medical instrument, which is manipulatable through a linkage in the field of view of the rigid viewing tube of the endoscope, characterized in that an operating sleeve (4) is provided concentrically round the viewing tube (5), that several concentric, resilient gripping arms (1, 2, 3) are fixed to the end of the operating sleeve (4) and the latter is arranged in a concentric and axially displaceable manner in an endoscope shaft in such a way that the gripping arm can be drawn into the latter and laterally resiliently moved out of the latter.

2. Endoscope according to claim 1, characterized in that the linkage (7, 8) for the movement of the operating sleeve (4) is so connected by means of a straight movement lever bar (9, 10) and an axially movable carriage (14) to the viewing tube (5), that the displacement of the latter takes place to a different extent as compared with the operating sleeve displacement.

3. Endoscope according to claim 1, characterized in that the operating sleeve (4) is connected to one or more operating elements (11, 12, 15, 16), which pass through the shaft closure (13) in a sliding guide and are detachably fixed in the axially movable carriage (14) of the linkage.

4. Endoscope according to claim 1, characterized in that the operating elements are constructed as wires (11, 12), which are soldered to the operating sleeve (4).

5. Endoscope according to claim 4, characterized in that the two wires (11, 12) for guidance purposes in the schaft closure (13) are separated from one another in fork-shaped manner prior to the entry of the latter.

6. Endoscope according to claim 3, characterized in that the operating elements are constructed as one or more one-piece extensions (15, 16) of the operating sleeve (4) and having a smaller cross-section than the latter.

7. Endoscope according to claim 1, characterized in that an outer sleeve (19) enveloping the operating sleeve (4) is connected by fastening elements (21, 22) to the shaft closure (13).

8. Endoscope according to claim 1, characterized in that there is a through, fixed sleeve (20) for guiding the viewing tube (5), which terminates at a distance (b) upstream of the distal end of the outer sleeve (19).

## Revendications

1. Endoscope avec un instrument médicinal susceptible d'être manipulé dans la zone de vision du tube rigide de vision de l'endoscope par une tringlerie, endoscope caractérisé en ce qu'il est prévu, concentriquement autour du tube de vision (3), une douille de manœuvre (4), et qu'à l'extrémité de cette douille de manœuvre (4) sont fixés plusieurs bras élastiques de préhension (1, 2, 3) disposés concentriquement les uns par rapport aux autres, tandis que la douille de manœuvre (4) est disposée de façon à pouvoir coulisser concentriquement et axialement à l'intérieur d'un fût d'endoscope (6), de sorte que les bras de préhension peuvent être retirés dans le fût (6) de l'endoscope et poussés hors de celui-ci en s'écartant latéralement de façon élastique.

2. Endoscope selon la revendication 1, caractérisé en ce que la tringlerie (7, 8) permettant de déplacer la douille de manœuvre (4) est reliée au tube de vision (5) par l'intermédiaire d'une tringlerie à leviers de poussée graduée (9, 10) et d'un chariot (14) susceptible d'être déplacé axialement, de sorte que le coulissement du tube de vision (5) s'effectue selon une mesure différente par rapport au coulissement de la douille de manœuvre.

3. Endoscope selon la revendication 1, caractérisé en ce que la douille de manœuvre (4) est reliée à un ou plusieurs éléments de manœuvre (11, 12; 15, 16) qui traversent la fermeture (13) du fût dans un guidage lisse et qui sont fixés de façon amovible sur le chariot (14), susceptible d'être déplacé axialement de la tringlerie.

4. Endoscope selon la revendication 1, caractérisé en ce que les éléments de manœuvre revêtent la forme de fils métalliques (11, 12) qui sont soudés à la douille de manœuvre (4).

5. Endoscope selon la revendication 4, caractérisé en ce que les deux fils métalliques (11, 12) pour assurer leur guidage dans la fermeture (13) du fût, sont séparés l'un de l'autre en forme de fourche avant leur entrée dans cette fermeture.

6. Endoscope selon la revendication 3, caractérisé en ce que les éléments de manœuvre revêtent la forme d'un ou plusieurs prolongements (15, 16) de la douille de manœuvre (4), d'un seul tenant avec celle-ci et ayant une section transversale plus faible que cette douille.

7. Endoscope selon la revendication 1, caractérisé en ce qu'une douille externe (19), enveloppant la douille de manœuvre (4), est reliée à la fermeture (13) du fût par des éléments de fixation (21, 22).

8. Endoscope selon la revendication 1, caractérisé en ce qu'il est prévu une douille fixe traversante (20) pour le guidage du tube de vision (5), cette douille se terminant à une certaine distance (b) avant l'extrémité distale de la douille externe (19).

FIG.1

FIG.2

EP 0 117 894 B1

FIG.3

FIG.4

FIG.5

FIG.6